(19) Europäisches Patentamt
European Patent Office
Office européen des brevets

(11) **EP 4 393 905 A1**

(12) **EUROPEAN PATENT APPLICATION**
published in accordance with Art. 153(4) EPC

(43) Date of publication:
**03.07.2024 Bulletin 2024/27**

(21) Application number: **22861456.6**

(22) Date of filing: **26.08.2022**

(51) International Patent Classification (IPC):
***C07C 51/367*** *(2006.01)* ***C07B 61/00*** *(2006.01)*
***C07C 51/47*** *(2006.01)* ***C07C 65/10*** *(2006.01)*

(52) Cooperative Patent Classification (CPC):
**C07B 61/00; C07C 51/367; C07C 51/47; C07C 65/10**

(86) International application number:
**PCT/JP2022/032190**

(87) International publication number:
**WO 2023/027168 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2021 JP 2021139134**

(71) Applicant: **API Corporation**
**Chikujo-gun, Fukuoka 871-0801 (JP)**

(72) Inventors:
- **TANIIKE, Hirotsugu**
  **Chikujo-gun, Fukuoka 871-0801 (JP)**
- **TSUJI, Shunsuke**
  **Chikujo-gun, Fukuoka 871-0801 (JP)**
- **NAGAHAMA, Masaki**
  **Chikujo-gun, Fukuoka 871-0801 (JP)**
- **KOBA, Yurie**
  **Chikujo-gun, Fukuoka 871-0801 (JP)**

(74) Representative: **Godemeyer Blum Lenze Patentanwälte Partnerschaft mbB - werkpatent**
**An den Gärten 7**
**51491 Overath (DE)**

(54) **SALICYLIC ACID PRODUCTION METHOD**

(57) Salicylic acid is produced with high productivity by reacting 2-halogenated benzoic acid in an aqueous solvent at a reaction temperature of 155°C or higher and 300°C or lower in the presence of a copper source and a ligand. Preferably, the method further includes a purification step A in which the obtained salicylic acid is brought into contact with a styrene-based synthetic adsorbent and/or a purification step B in which the obtained salicylic acid is brought into contact with zeolite. The compound is led to raw materials for various pharmaceuticals or intermediates thereof (for example, acetylsalicylic acid).

[Fig. 1]

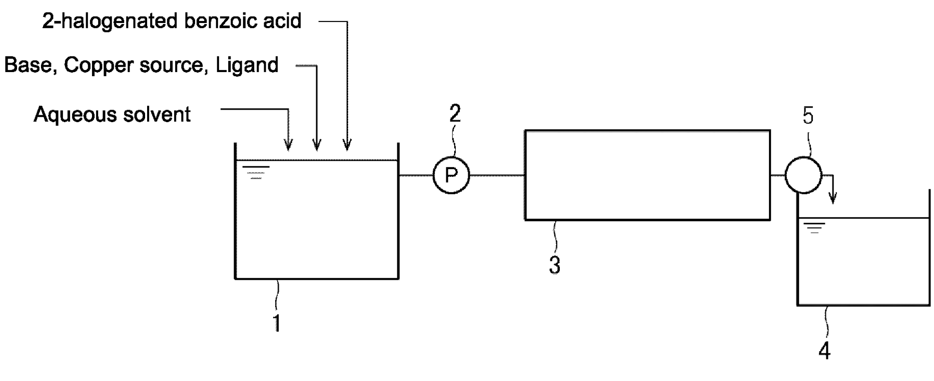


EP 4 393 905 A1

## Description

### Technical Field

The present invention relates to a method for producing salicylic acid, which is useful as a raw material or intermediate for synthesizing various pharmaceuticals, agricultural chemicals, chemical products, etc.

### Background Art

Salicylic acid is useful as a raw material or intermediate for synthesizing various pharmaceuticals, agricultural chemicals, chemical products, etc. For example, acetylsalicylic acid that is a derivative of salicylic acid is widely used as an antipyretic analgesic, and methyl salicylate is widely used as an anti-inflammatory analgesic.

A production method using a Kolbe-Schmitt reaction has been known as a method for producing salicylic acid. For example, Patent Literature 1 discloses a method for producing salicylic acid by reacting sodium phenolate and carbon dioxide under high temperature and high pressure conditions according to the following reaction formula.

[Chem. 1]

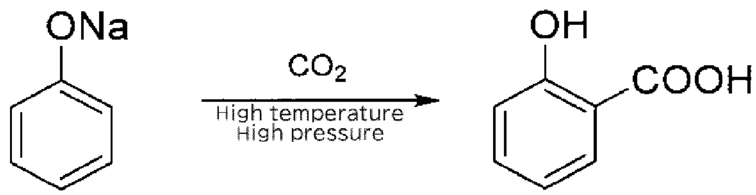


In recent years, a method for producing salicylic acid from 2-chlorobenzoic acid was disclosed. For example, Non-Patent Literature 1 discloses a method for producing salicylic acid in a yield of 91% by reacting 2-chlorobenzoic acid in the presence of potassium carbonate, copper powder, and pyridine for 2 hours while heating and refluxing it in an aqueous solvent according to the following reaction formula.

[Chem. 2]

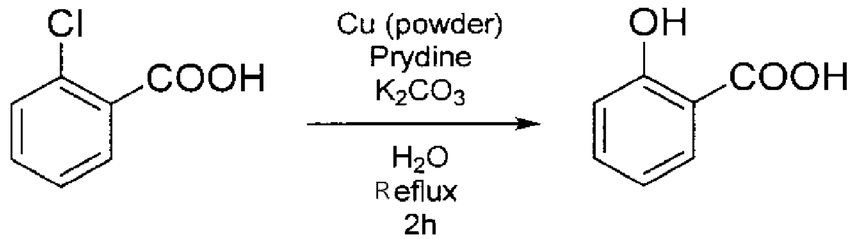


A method for producing salicylic acid from 2-chlorobenzoic acid has been known. For example, Patent Literature 2 discloses a method for producing salicylic acid in which 2-bromobenzoic acid is reacted at 100°C for 3 hours in an aqueous solvent in the presence of sodium carbonate, copper(I) bromide and N,N'-dimethylcyclohexane-1,2-diamine according to the following reaction formula, to obtain salicylic acid at a yield of 85%.

[Chem. 3]

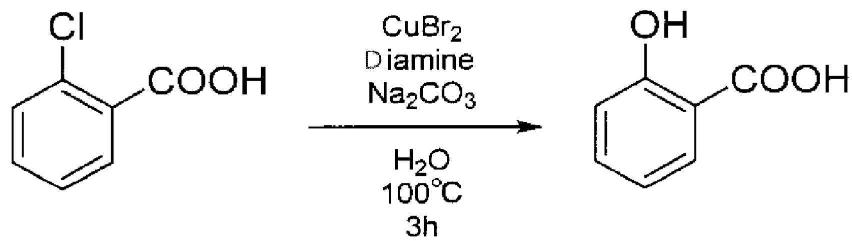

The methods described in Non-Patent Literature 1 and Patent Literature 2 require a long reaction time, and therefore it is desired a more productive and industrially advantageous production method, that is an industrial production method with high productivity, low cost, and stable production.

Furthermore, salicylic acid obtained by these methods is considered to contain about several percent of by-products such as aromatic compounds or the like, as inferred from the production method and yield.

In general, raw materials and intermediates for pharmaceutical synthesis are required to have high purity to avoid unexpected side effects caused by impurities contained in them. Examples of impurities include by-products generated during their production. In some cases, by-products can be removed during the purification or manufacturing process of the target drug. However, when pharmaceuticals are manufactured industrially and in large quantities, it is desired to further reduce manufacturing costs and purification costs in order to ensure a stable supply of pharmaceuticals, suppress prices, and the like. Therefore, raw materials and intermediates for synthesis of pharmaceuticals having fewer impurities and higher purity are desired.

For this reason, it is desirable to produce salicylic acid having a lower content of by-products that become impurities.

## Citation List

### Patent Literature

[Patent Literature 1] JP S58-15939 A
[Patent Literature 2] JP 2010-511043 A

### [Non-patent Literature]

[Non-patent Literature 1] SYNTHETIC COMMUNICATIONS, 32(13), 2055-2059 (2002)

## Summary of Invention

### Technical Problem

An object of the present invention is to provide a method for producing salicylic acid with high productivity, low cost, and stable production.

Another object of the present invention is to provide a method for producing salicylic acid with a low content of by-products that become impurities.

### Solution to Problem

The present inventors have found that salicylic acid can be produced stably at low cost with high productivity by reacting 2-halogenated benzoic acid in an aqueous solvent in the presence of a copper source, a ligand, and a base under specific temperature conditions.

The present inventors have also found that salicylic acid having a low content of by-products than conventionally known methods can be produced by this method.

The gist of the present invention is as follows.

[1] A method for producing salicylic acid comprising a hydroxylation step in which salicylic acid is produced by reacting 2-halogenated benzoic acid in an aqueous solvent at a reaction temperature of 155°C or higher and 300°C or lower in the presence of a copper source, a ligand, and a base.
[2] The method for producing salicylic acid according to [1], wherein a reaction pressure of the reaction is 0.1 MPa or more and 10 MPa or less.
[3] The method for producing salicylic acid according to [1] or [2], wherein the ligand is a compound having one or more amino groups which is optionally substituted.
[4] The method for producing salicylic acid according to any one of [1] to [3], wherein the copper source is a copper compound.
[5] The method for producing salicylic acid according to any one of [1] to [4], comprising a purification step B in which the salicylic acid produced in the hydroxylation step is brought into contact with zeolite.
[6] The method for producing salicylic acid according to any one of [1] to [5], comprising a purification step A in

which the salicylic acid produced in the hydroxylation step is brought into contact with a synthetic adsorbent.

[7] The method for producing salicylic acid according to any one of [1] to [6], wherein the reaction is a flow synthesis reaction.

[8] The method for producing salicylic acid according to [6] or [7], the salicylic acid from the purification step A or the purification step B has HPLC purity of 95 area % or more, and contains one or more aromatic compounds represented by the following formulas (a) to (g) as impurities, and the content of each aromatic compound is 0.5 area % or less.

[Chem. 4]

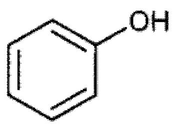

(a)

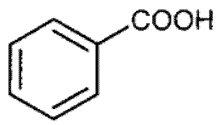

(b)

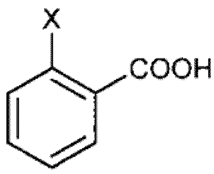

(c)

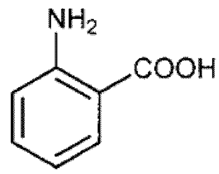

(d)

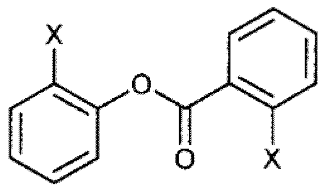

(e)

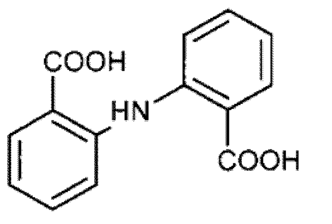

(f)

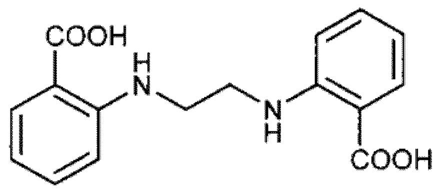

(g)

Advantageous Effects of Invention

According to the method for producing salicylic acid of the present invention, salicylic acid having low byproduct content can be produced stably with high productivity at low cost.

According to the present invention, salicylic acid having low impurity content can be produced. Salicylic acid produced according to the present invention is useful as a raw material or intermediate for synthesizing various pharmaceuticals, agricultural chemicals, chemical products, etc. In particular, due to its high purity, it is useful as a raw material for the synthesis of various pharmaceuticals or their intermediates, such as acetylsalicylic acid.

Brief Description of Drawing

[Fig. 1] Fig. 1 is a system diagram of a flow synthesis reactor conducting the method for producing salicylic acid according to an embodiment of the present invention. Description of Embodiments

Hereinafter, embodiments of the method for producing salicylic acid of the present invention will be described in detail.

The method for producing salicylic acid of the present invention includes a hydroxylation step in which salicylic acid is produced by reacting 2-halogenated benzoic acid in an aqueous solvent at a reaction temperature of 145°C or higher and 300°C or lower in the presence of a copper source, a ligand, and a base.

The method for producing salicylic acid of the present invention may include a purification step A in which the salicylic acid obtained in the hydroxylation step is brought into contact with a synthetic adsorbent, and/or a purification step B in which the salicylic acid obtained in the hydroxylation step is brought into contact with zeolite.

[Hydroxylation step]

The method for producing salicylic acid of the present invention is a method for producing salicylic acid by hydrolyzing 2-halogenated benzoic acid in an aqueous solvent in the presence of a copper source, a ligand, and a base.

An embodiment the present invention includes a method in which 2-halogenated benzoic acid, an aqueous

solvent, a base, a copper source, and a ligand are mixed in a batch-type synthesis reactor and reacted the mixture under reaction conditions. Another embodiment of the present invention includes a method using a flow synthesis reactor shown in Fig. 1, in which a mixed solution of 2-halogenated benzoic acid, a base, an aqueous solvent, a copper source, and a ligand prepared in a preparation tank 1 is continuously pumped by a pump 2, and 2-halogenated benzoic acid continuously hydrolyzed in a flow synthesis reactor 3 under reaction conditions and a reaction solution containing salicylic acid flowing out from the flow synthesis reactor 3 is collected in a collection tank 4.

The method of the present invention is preferably carried out by flow synthesis reaction using a flow synthesis reactor from the viewpoint of ease of control of reaction temperature and reaction pressure and operability. Details of this flow synthesis reactor will be described later.

<2-halogenated benzoic acid>

As the 2-halogenated benzoic acid, at least one selected from 2-chlorobenzoic acid, 2-bromobenzoic acid, and 2-iodobenzoic acid can be used. The 2-halogenated benzoic acid may be a commercially available product or one obtained by a known method or a method analogous thereto.

As 2-halogenated benzoic acid, 2-chlorobenzoic acid is preferred from the viewpoints of cost and reactivity.

<Base>

Examples of a base include inorganic bases and organic bases. It is preferable to use an inorganic base in order to enhance the reactivity in an aqueous solvent, which will be described later. Although two or more types of bases may be used in any ratio, it is preferable to use one type alone from the viewpoints of cost and reactivity.

Examples of the inorganic base include sodium carbonate, sodium hydrogen carbonate, potassium carbonate, potassium hydrogen carbonate, sodium hydroxide, or potassium hydroxide and the like. Among these, from the viewpoints of cost and reactivity, sodium carbonate or potassium carbonate is preferred, and sodium carbonate is particularly preferred.

The organic base is not particularly limited as long as the reaction proceeds, but examples thereof include tertiary alkylamines such as triethylamine, pyridine and the like.

The amount of the base to be used is usually 1 mol or more as a lower limit, and usually 10 mol or less as an upper limit, and preferably 5 mol or less, particularly preferably 2 mol or less, from the viewpoint of cost, per 1 mol of 2-halogenated benzoic acid.

<Aqueous solvent>

The aqueous solvent is selected from water or a water-soluble organic solvent. These may be used alone or in combination of two or more in any proportion as long as they do not adversely affect the reaction.

In the present invention, it is preferable to use water substantially alone from the viewpoints of cost, reactivity, and environmental load reduction. Here, "using water substantially alone" means that the ratio of water in the solvent is 95% by mass or more. By using water substantially alone, the reaction can be carried out in a homogeneous system in a short time and efficiently.

The water-soluble organic solvent may be any solvent as long as it does not inhibit the hydroxylation of 2-halogenated benzoic acid, and for example, tetrahydrofuran, dioxane and the like can be used.

From the viewpoint of productivity and reactivity, the lower limit of the amount of the aqueous solvent is usually 1 L or more, preferably 2 L or more, more preferably 5 L or more, and particularly preferably 9 L or more per 1 kg of 2-halogenated benzoic acid. The upper limit is usually 30 L or less, preferably 25 L or less, more preferably 20 L or less, and particularly preferably 18 L or less.

By controlling the amount of the aqueous solvent within the above range, the fluidity of the solution is improved and the reaction can be carried out efficiently.

In the present invention, it is usually preferable that the 2-halogenated benzoic acid used as a raw material is dissolved in an aqueous solvent and the reaction proceeds as a homogeneous solution, but if the reaction proceeds, it may be in slurry form without being completely dissolved.

<Copper source>

In the method for producing salicylic acid of the present invention, salicylic acid can be produced with high productivity by using a copper source.

As the copper source, copper alone or a copper compound such as copper halide, copper oxide, copper inorganic acid salt, or copper organic acid salt and the like can be used. Among these, from the viewpoints of cost,

reactivity, and solubility in an aqueous solvent, copper compounds are preferred, copper halides, copper inorganic acid salts, or copper organic acid salts are more preferred, and copper halides are particularly preferred.

As the copper source, an anhydride or a hydrate may be used as long as the reaction proceeds.

Examples of the copper halide include copper chloride such as CuCl or $CuCl_2$, copper bromide such as CuBr or $CuBr_2$ and copper iodide such as CuI. Among these, from the viewpoints of cost and reactivity, copper chloride is preferred, and $CuCl_2$ is particularly preferred.

Examples of the copper oxide include $Cu_2O$ or CuO.

Examples of the copper inorganic acid salt include $CuSO_4$, copper(II) nitrate, copper(II) carbonate, or copper(II) hydroxide and the like.

Examples of the copper organic acid salt include copper (II) formate, copper (II) citrate, copper (II) gluconate, copper (II) acetate, or copper (I) acetate and the like.

The amount of the copper source may be any amount that allows the reaction to proceed, but is usually 1 mol or more per 1 mol of 2-halogenated benzoic acid.

In the present invention, by using the ligand described below, the reaction can be performed using a catalytic amount of the copper source. When a copper source is used together with a ligand, the amount of the copper source used is usually 0.0001 mol or more and 1 mol or less, preferably 0.001 mol or more and 0.5 mol or less per 1 mol of 2-halogenated benzoic acid. By reducing the amount of the copper source used, the cost and copper content of the salicylic acid produced can be reduced.

<Ligand>

In the present invention, by using a ligand together with a copper source, the amount of the copper source can be reduced to a catalytic amount, and salicylic acid can be produced at low cost and with high productivity.

It is thought that the ligand forms a complex by reacting with the copper source, and the complex functions as a catalyst. Examples of known complex of the copper source and the ligand include copper amine complexes such as copper ethylenediamine complex and the like; copper amino acid complexes such as copper histidine complex and the like.

The ligand is preferably one that increases the activity of the copper source and promotes the progress of the reaction, and amines are usually used. Here, amine means a compound having one or more amino groups that may be substituted.

Examples of amine include aliphatic amines, heterocyclic amines, and amine derivatives. From the viewpoint of cost and reactivity, aliphatic amines are preferred.

The lower limit of the carbon number of the amine used in the present invention is usually 1 or more, preferably 2 or more, and the upper limit is usually 12 or less, preferably 8 or less, more preferably 6 or less, and particularly preferably 4 or less.

Examples of the aliphatic amine include monoamines such as dimethylamine or diethylamine and the like; diamines such as ethylenediamine, N,N'-dimethylethylenediamine, N,N'-tetramethylethylenediamine, 1,3-propyldiamine, or N,N'-dimethylcyclohexane-1,2-diamine and the like; triamines such as spermidine and the like; tetraamines such as triethylenetetramine, N,N'-bis(2-aminoethyl)-1,3-propanediamine, or N,N'-bis(3- (aminopropyl)-1,4-butanediamine (spermine) and the like.

Among these, from the viewpoints of cost, reactivity, and impurity suppression, diamine is preferred, and ethylenediamine is particularly preferred.

Examples of the heterocyclic amine include pyridine, piperidine, dipyridyl, 2,2'-dipyridylamine, and 1,10-phenanthroline.

Examples of the amine derivative include iminodiacetic acid, 4-aminobutyric acid, L-histidine, N-methyl-L-proline, L-proline, L-valine, L-aspartic acid, L-serine, L-lysine, N-methyl- L-alanine, L-alanine, L-phenylalanine, D-methionine, ethylenediaminetetraacetic acid, or β-alanine and the like.

The amount of the ligand may vary depending on the type of the copper source and the type of ligand. The lower limit of the amount of the ligand is usually 0.05 mol or more, preferably 1 mol or more, and the upper limit is usually 300 mol or less, preferably 200 mol or less, per 1 mol of the copper source.

When the amount of the ligand is too small, the reaction may not proceed efficiently and the amount of copper source used may not be reduced. When the amount of the ligand used is too large, the amount of by-products may increase.

<Reaction conditions>

(Reaction temperature)

The lower limit of the reaction temperature is usually 155°C or higher, preferably 165°C or higher, more preferably 175°C or higher, even more preferably 185°C or higher, and particularly preferably 195°C or higher, from the viewpoint of reactivity and productivity. The upper limit of the reaction temperature is not particularly limited as long as the reaction proceeds, but is usually 300°C or lower, preferably 250°C or lower, more preferably 240°C or lower, even more preferably 230°C or lower, and particularly preferably 220°C or lower.

When the reaction temperature is too low, reactivity may decrease. When the reaction temperature is too high, decomposition of the complex or side reactions may occur, resulting in an increase in impurities and a decrease in productivity and purity of the target product.

(Reaction pressure)

The reaction can be carried out under normal pressure or increased pressure, but when the reaction is carried out under high temperature conditions higher than the boiling point of the solvent, it is preferable to apply pressure to achieve the desired high temperature conditions. The lower limit of the reaction pressure is usually 0.1 MPa or more, preferably 0.2 MPa or more, more preferably 0.3 MPa or more, and particularly preferably 0.4 MPa or more. The upper limit of the reaction pressure is usually 10 MPa or less, preferably 8 MPa or less, more preferably 6 MPa or less, and particularly preferably 4 MPa or less.

When using a batch reactor, the reaction can be efficiently carried out at the desired reaction pressure by adjusting the pressure using a pressure-resistant vessel.

When using a flow reactor, the reaction can be efficiently carried out at the desired reaction pressure by applying back pressure to the flow path using a back pressure valve or the like to adjust the pressure.

For example, when water is used as the aqueous solvent, it is preferably that the reaction pressure should be 0.54 MPa or more to increase the reaction temperature of 155°C or higher, 0.70 MPa or more to increase the reaction temperature of the 165°C or higher, 0.89 MPa or more to increase the reaction temperature of 175°C or higher, 1.12 MPa or more to increase the reaction temperature of 185°C or higher, 1.56 MPa or more to increase the reaction temperature of 200°C or higher, 2.32 MPa or more to increase the reaction temperature of 220°C or higher, 2.80 MPa or more to increase the reaction temperature of 230°C or higher, 3.35 MPa or more to increase the reaction temperature of 240°C or higher, 3.98 MPa or more to increase the reaction temperature of 250°C or higher, 5.51 MPa or more to increase the reaction temperature of 270°C or higher, 8.59 MPa or more to increase the reaction temperature of 300°C or higher.

(Reaction time)

The reaction time means the time that the raw-material mixture (a mixture of 2-halogenated benzoic acid, a base, a copper source, a ligand, and an aqueous solvent) stays in the reactor used in the present invention. Preferred reaction time varies depending on the reaction temperature and reaction pressure. The reaction time is usually 0.1 minutes or more and 60 minutes or less, preferably 0.5 minutes or more and 30 minutes or less, and particularly preferably 1 minute or more and 10 minutes or less in order to improve the amount of salicylic acid produced per unit time.

(Reaction process)

In the present invention, 2-halogenated benzoic acid is reacted with a complex formed by a copper source and a ligand in the presence of a base in an aqueous solvent. The reaction method is not particularly limited, but as mentioned above, it is industrially preferable to use a flow synthesis reactor (flow type reactor). That is, as shown in Fig.1, a mixture of 2-halogenated benzoic acid, a base, a copper source, a ligand, and an aqueous solvent is prepared in preparation tank 1, fed to flow synthesis reactor (flow type reactor) 3 by pump 2, heated in flow synthesis reactor 3 to carry out the reaction, and the reaction liquid is collected in collection tank 4.

(Reactor)

The reactor is not particularly limited as long as the reaction proceeds, but examples thereof include flow type reactors and batch type synthesis reactors.

As a batch type synthesis reactor, one equipped with a channel for introducing and discharging substrates and the like, a jacket capable of controlling the reaction temperature, a device capable of controlling the reaction pressure,

and a stirrer and the like can be used.

The flow synthesis reactor is preferably tubular, and the tubular shape may be straight, curved, or spiral. From the viewpoint of reactor volume, a spiral flow synthesis reactor is particularly preferred.

The size of the flow synthesis reactor is selected according to the scale of production. For example, the inner diameter is 1 mm or more and 50 mm or less, and the length is selected according to the desired residence time. The flow synthesis reactor may be equipped with a temperature control mechanism.

The introduction and discharge of substrates and the like into the flow synthesis reactor can be carried out quantitatively by liquid feeding using a syringe pump, cylinder pump, diaphragm pump, plunger pump or the like. A back pressure valve that can control the reaction pressure or an in-line analysis device may be provided in the flow path on the outflow side of the reaction liquid from the flow synthesis reactor.

(Heating device)

In the hydroxylation step, the reaction solution can be heated using a hot water bath, oil bath, or microwave and the like, although there are no particular limitations as long as the reaction temperature can be controlled.

[Post- processing]

The target salicylic acid can be isolated from the obtained reaction solution by mixing the obtained reaction solution with acid, precipitating salicylic acid, and obtaining crystals of salicylic acid by solid-liquid separation (crystallization step described later). The obtained crystals may be further purified by known purification means such as recrystallization or column chromatography, or purified by a purification step using an adsorbent, for example, purification step A and/or purification step B described below.

Furthermore, after the salicylic acid solution obtained in the hydroxylation step is purified in the purification step A and/or purification step B described below, it may be further purified by known purification means such as recrystallization or column chromatography and the like.

In the purification process according to the present invention, the salicylic acid purified in the purification step A described below may be further purified in the purification step B described below, or the salicylic acid purified in the purification step B may be further purified in the purification step A. From the viewpoint of impurity removal efficiency and industrial productivity, it is preferable that the salicylic acid purified in the purification step A is further purified in the purification step B.

<Adsorbent>

As the adsorbent used in the purification process according to the present invention, one or more of zeolite, synthetic adsorbent, or ion exchange resin can be used.

(Zeolite)

In the present invention, zeolite means an aluminosilicate containing an alkali metal and/or an alkaline earth metal. It is known that the higher the $SiO_2/Al_2O_3$ ratio of zeolite, the more hydrophobic the zeolite.

The lower limit of the $SiO_2/Al_2O_3$ ratio of the zeolite used in the present invention is usually 10 or more, preferably 100 or more, more preferably 500 or more, even more preferably 1000 or more, particularly preferably 1500 or more, and the upper limit is usually 10,000 or less, preferably 4,000 or less, more preferably 3,000 or less, even more preferably 2,500 or less, particularly preferably 2,000 or less.

By using the zeolite having the above-mentioned $SiO_2/Al_2O_3$ ratio, the aromatic compound represented by the formula (b) described below can be efficiently removed.

The BET specific surface area of zeolite is usually 100 $m^2/g$ or more and 1000 $m^2/g$ or less, and preferably 200 $m^2/g$ or more and 500 $m^2/g$ or less, for the purpose of improving reactivity.

The most frequent pore radius of zeolite is usually 0.1 nm or more and 10 nm or less, preferably 0.3 nm or more and 5 nm or less, and particularly preferably 0.5 nm or more and 1 nm or less, for the purpose of improving the removal efficiency of impurities.

The BET specific surface area and the most frequent pore radius of zeolite can be measured by the nitrogen gas adsorption method according to the usual method.

The shape of the zeolite may be any shape as long as it can be in contact with the salicylic acid solution, and powder, pellet, membrane, or cylinder form can be used. Among these, from the viewpoint of impurity removal efficiency, particulate zeolite having a large contact area with the salicylic acid solution is preferred.

The particle diameter of the particulate zeolite is usually in the range of 0.01 $\mu$m or more and 100 $\mu$m or less,

and from the viewpoint of industrial handling or the like, preferably 0.1 μm or more and 100 μm or less, particularly preferably 1 μm or more and 50 μm or less.

The particle size of the zeolite is the average particle size measured according to a conventional method using a laser diffraction particle size distribution measurement method.

As the zeolite, for example, commercially available products such as HSZ (registered trademark)-900, HSZ (registered trademark)-891HOA, HSZ (registered trademark)-800, HSZ (registered trademark)-700, HSZ (registered trademark)-600, HSZ (registered trademark)-500 or HSZ (registered trademark)-300 manufactured by Tosoh Corporation and the like can be used. Among these, from the viewpoint of impurity removal efficiency, HSZ (registered trademark)-891HOA or HSZ (registered trademark)-800 is preferred, and HSZ (registered trademark)-891HOA is particularly preferred.

(Synthetic adsorbent)

In the present invention, a synthetic adsorbent is a porous synthetic adsorbent made of a porous organic polymer produced by chemical synthesis.

The base material of the synthetic adsorbent used in the present invention includes an aromatic, substituted aromatic, or acrylic polymer or copolymer (hereinafter, "polymer or copolymer" is referred to as "(co)polymer").

Examples of the aromatic (co)polymer include styrene-divinylbenzene copolymer and divinylbenzene polymer.

Examples of substituted aromatic (co)polymers include bromostyrene-divinylbenzene copolymer.

Examples of the acrylic (co)polymer include methacrylic ester (co)polymers such as methyl methacrylate-bis(methacrylic acid) ethylene glycol copolymer.

Among these, aromatic (co)polymers are preferred from the viewpoint of insolubility in organic solvents and stability in acidic and alkaline solutions, and styrene-divinylbenzene based copolymers such as styrene-divinylbenzene copolymer and bromostyrene-divinylbenzene copolymer are more preferred, and styrene-divinylbenzene copolymer is particularly preferred.

The synthetic adsorbent used in the present invention is preferably one that does not substantially have a functional group such as an ion exchange group, for example, one that has an ion exchange capacity of less than 1 meq/g or one that is nonpolar.

The pore volume of the synthetic adsorbent used in the present invention is usually 0.1 mL/g or more and 3 mL/g or less, preferably 0.5 mL/g or more and 2 mL/g or less, and particularly preferably 1 mL/g or more and 1.5mL/g or less for the purpose of improving reactivity.

The BET specific surface area of the synthetic adsorbent is usually 200 $m^2/g$ or more and 2000 $m^2/g$ or less, preferably 300 $m^2/g$ or more and 1500 $m^2/g$ or less, more preferably 400 $m^2/g$ or more and 1000 $m^2/g$ or less, and particularly preferably 500$m^2/g$ or more and 700$m^2/g$ or less, for the purpose of improving reactivity.

The most frequent pore radius of the synthetic adsorbent is usually 1 nm or more and 50 nm or less, preferably 5 nm or more and 40 nm or less, and particularly preferably 10 nm or more and 30 nm or less, for the purpose of improving reactivity. By using a synthetic adsorbent within the above range, it is possible to efficiently remove one or more aromatic compounds selected from the aromatic compounds represented by formulas (f), (g), and (e) described below.

The pore volume, BET specific surface area and most frequent pore radius of the synthetic adsorbents can be measured by the nitrogen gas adsorption method according to the usual method.

The shape and size of the synthetic adsorbent are not particularly limited as long as it can be packed into a column and do not hinder the flow of the salicylic acid solution. As the synthetic adsorbent, particles, pellets, membranes, and cylinders can be used, but from the viewpoint of ease of packing, particle-shaped adsorbents are preferred.

The particle diameter of the particulate synthetic adsorbent is usually in the range of 1 μm or more and 2000 um or less, and preferably in the range of 3 μm or more and 2000 μm or less. From the viewpoint of industrial handling and the like, the particle diameter of the synthetic adsorbent is preferably in the range of 4 μm or more and 1000 μm or less, and the most frequent particle size is 50 um or more, preferably 150 μm or more, and particularly preferably 250 um or more.

The particle size of the synthetic adsorbent is the average particle size measured by the laser diffraction particle size distribution method according to the usual method.

Examples of the synthetic adsorbents include commercial products such as DIAION (registered trademark) HP20SS, HP20, HP21, HP2MG, HP2MGL, SEPABEADS (registered trademark) SP20SS, SP70, SP207, SP700, SP850, XAD™-2, XAD™4, XAD™1600N or XAD™7HP manufactured by Mitsubishi Chemical Corporation and the like. Among these, DIAION (registered trademark) HP21 or XAD™4 is preferred from the viewpoint of impurity removal efficiency.

Table 1 shows the physical properties of these commercially available synthetic adsorbents.

[Table 1]

| Product name | Structure | BET specific surface area **[$m^2$/g]** | Pore volume **[mL/g]** | Most frequent pore radius **[nm]** | Most frequent particle size **[μm]** |
|---|---|---|---|---|---|
| SEPABEADS **SP2DSS** | Styrene-divinylbenzene copolymer | **560** | **1. 2** | **29** | **63** or more |
| DIAION **HP20SS** | Styrene-divinylbenzene copolymer | **560** | **1. 2** | **29** | **63** or more |
| DIAION **HP20** | Styrene-divinylbenzene copolymer | **590** | **1. 3** | **29** | **250** or more |
| DIAION **HP21** | Styrene-divinylbenzene copolymer | **640** | **1. 3** | **11** | **250** or more |
| AMBERLITE **XAD™4** | Styrene-divinylbenzene copolymer | **880** | **1. 3** | **12** | **490** or more |
| AMBERLITE **XAD™7HP** | Acrylic polymer | **500** | **0. 6** | **10** | **430** or more |

(Ion exchange resin)

In the present invention, an ion exchange resin is a synthetic adsorbent having an ion exchange group.

The ion exchange group can be anything that adsorbs copper ions, but is usually a cation exchange group. As the cation exchange group, an iminodiacetic acid group is preferred.

The pore volume of the ion exchange resin used in the present invention is usually 0.1 mL/g or more and 4 mL/g or less, preferably 0.5 mL/g or more and 3 mL/g or less, and particularly preferably 1 mL or more and 2mL/g or less, for the purpose of improving impurity removal efficiency.

The BET specific surface area of the ion exchange resin is usually 200 $m^2$/g or more and 2000 $m^2$/g or less, preferably 300 $m^2$/g or more and 1500 $m^2$/g or less, and particularly preferably 400 $m^2$/g or more and 1000 $m^2$/g or less, for the purpose of improving impurity removal efficiency.

The most frequent pore radius of the ion exchange resin is usually 1 nm or more and 100 nm or less, preferably 5 nm or more and 50 nm or less, and particularly preferably 10 nm or more and 25 nm or less, for the purpose of improving impurity removal efficiency.

By using the ion exchange resin described above, copper ions during the reaction can be efficiently removed.

The pore volume, BET specific surface area, and most frequent pore radius of the ion exchange resin can be measured by the nitrogen gas adsorption method according to the usual method.

The shape and size of the ion exchange resin are not particularly limited as long as it can be packed into a column and do not hinder the flow of the salicylic acid solution. As the ion exchange resin, particles, pellets, membranes, and cylinders can be used, but from the viewpoint of ease of packing, particle-shaped ion exchange resins are preferred.

The harmonic mean diameter of the particulate ion exchange resin is usually in the range of 0.1 mm or more and 1 mm or less, and preferably in the range of 0.3 mm or more and 0.8 mm or less, from the viewpoint of industrial handling and the like.

The particle size of the ion exchange resin is the average particle size measured by the laser diffraction particle size distribution method according to the usual method.

As the ion exchange resin, commercially available products such as DIAION (registered trademark) CR11, CR20, CRB03, CRB05 manufactured by Mitsubishi Chemical Corporation, AMBERSEP™IRC748 and AMBERSEP™IRC747UPS manufactured by Organo Corporation and the like can be used. Among these, CR11 is preferred from the viewpoint of copper ion removal efficiency.

<Other means of purification>

In addition to the ion exchange resin described above, copper in the reaction solution may be removed by the known method of precipitating copper using a reducing agent such as hydrosulfite or hydrazine and the like.
Another method is to use activated carbon to adsorb and remove impurities from the reaction solution.
As activated carbon, for example, Shirasagi, Kyoryoku Shirasagi, Seisei Shirasagi and the like manufactured by Osaka Gas Chemicals Co., Ltd. may be used. Among these, Kyoryoku Shirasagi is preferred from the viewpoint of impurity removal efficiency.
The amount of activated carbon used is 1% by mass or more and 100% by mass or less, preferably 5% by mass or more and 50% by mass or less, and more preferably 5% by mass or more and 25% by mass or less, based on the 2-halogenated benzoic acid used in the reaction.

<Purification step A>

Purification step A is a step to reduce impurities in salicylic acid and increase the purity of salicylic acid by bringing the salicylic acid obtained in the hydroxylation step or the salicylic acid obtained in the purification step B described below into contact with a synthetic adsorbent.
In the purification step A, salicylic acid is brought into contact with the specific adsorbent described above to remove at least one of the aromatic compounds represented by the formulas (a) to (g) described below, particularly at least one or more selected from the aromatic compounds represented by the formulas (b), (f), (g) and (e).

(Salicylic acid)

The salicylic acid to be purified in the purification step A is usually in the form of a solution, preferably an aqueous solution. The solution can be a salicylic acid solution obtained in the above-mentioned hydroxylation step or in the purification step B and/or the purification step C described below.
It is sufficient that the salicylic acid contained in the salicylic acid solution is dissolved when the salicylic acid is in contact with the adsorbent. The concentration of salicylic acid in the salicylic acid solution is usually 0.1% by mass or more and 80% by mass or less, preferably 1% by mass or more and 75% by mass or less, and particularly preferably 5% by mass or more and 70% by mass or less, from the viewpoint of productivity and reactivity.

(Adsorbent)

Among the adsorbents mentioned above, in the purification step A, a styrene-based synthetic adsorbent is particularly used, preferably a styrene-divinylbenzene copolymer such as a styrene-divinylbenzene copolymer, a bromostyrene-divinylbenzene copolymer and the like is used, and particularly preferably a styrene-divinylbenzene copolymer is used. As mentioned above, styrene-based synthetic adsorbents that have substantially no functional groups such as ion-exchange groups, for example, those having ion-exchange capacity of less than 1 meq/g or non-polarity, are preferred because they have less effect on the reaction.
The most frequent pore radius of the styrene-based synthetic adsorbent used in the purification step A is usually 1 nm or more and 50 nm or less, preferably 2 nm or more and 45 nm or less, and particularly preferably 3 nm or more and 40 nm or less, for the purpose of improving reactivity. By using a styrene-based synthetic adsorbent in the above range, it is possible to efficiently remove aromatic compounds represented by the formulas (f), (g) and (e) described below.
The preferred pore volume, BET specific surface area, shape and other properties of the styrene-based synthetic adsorbent used in the purification step A are as described in the adsorbent section above.
As the styrene-based synthetic adsorbent used in the purification step A, for example, commercially available products such as DIAION (registered trademark) HP20SS, HP20, HP21, Sepabeads (registered trademark) SP20SS, SP700 manufactured by Mitsubishi Chemical Corporation and the like can be used. Among these, HP21 is preferred from the viewpoint of impurity removal efficiency.

(Purification conditions for purification step A)

<Contact temperature>

The contact temperature at which the salicylic acid solution and the styrene-based synthetic adsorbent are brought into contact is usually 5°C or higher and 100°C or lower, from the viewpoint of impurity removal efficiency and solubility, preferably 50°C or higher and 95°C or lower, and particularly preferably 60°C or higher and 90°C or lower.

<Contact time>

The contact time during which the salicylic acid solution and the styrene-based synthetic adsorbent are brought into contact is not particularly limited as long as impurities are sufficiently removed, but is usually 0.1 hours or more and 24 hours or less.

<Contact pressure>

The contact pressure at which the salicylic acid solution and the styrene-based synthetic adsorbent are brought into contact is usually normal pressure, but may be pressurized.

<pH>

The pH of the salicylic acid solution at which the salicylic acid solution and the styrene-based synthetic adsorbent are brought into contact may be within a range in which salicylic acid does not precipitate, and is usually pH 3 or more and pH 8 or less, from the viewpoint of impurity removal efficiency, preferably pH 3 or more and pH 7 or less, more preferably pH 3 or more and pH 6 or less, and particularly preferably pH 3 or more and pH 5 or less. By setting the pH in the above range, it is possible to efficiently remove at least one or more of the aromatic compounds selected from those represented by the formulas (b), (e), (f) and (g) described below.

(Post-processing)

Examples of the method for isolating the target salicylic acid from the salicylic acid solution obtained through purification step A include a method in which the obtained treated solution is mixed with a strong acid, salicylic acid is precipitated, and crystals of the salicylic acid are obtained by solid-liquid separation (crystallization step described below). The obtained crystals may be further purified by known purification methods such as recrystallization or column chromatography, or by the purification step B and/or the purification step C described below.

<Purification step B>

Purification step B is a step to reduce impurities in salicylic acid and increase the purity of salicylic acid by bringing the salicylic acid obtained in the hydroxylation step or the salicylic acid obtained in the aforementioned purification step A into contact with zeolite.

In the purification step B, salicylic acid is brought into contact with the adsorbent described above to remove at least one of the aromatic compounds represented by the formulas (a) to (g) below, particularly at least one or more selected from the aromatic compounds represented by the formulas (b), (e), (f) and (g).

(Salicylic acid)

As salicylic acid to be purified in the purification step B, a salicylic acid solution obtained in the above-mentioned hydroxylation step or in the purification step A described above can be used.

The concentration of the salicylic acid solution is usually 0.1% by mass or more and 80% by mass or less, preferably 1% by mass or more and 75% by mass or less, and particularly preferably 5% by mass or more and 70% by mass or less, from the viewpoint of productivity and reactivity.

(Adsorbent)

In the purification step B, the adsorbent described above can be used, but from the viewpoint of efficiently removing at least one kind selected from the aromatic compounds represented by the formulas (b), (e), (f) and (g), it is preferable to use the zeolite described above.

(Purification conditions for purification step B)

<Contact temperature>

The contact temperature at which the salicylic acid solution and the zeolite are brought into contact is usually 5°C or higher and 100°C or lower, preferably 50°C or higher and 95°C or lower, and particularly preferably 60°C or higher and 90°C or lower, from the viewpoint of impurity removal efficiency and solubility.

<Contact time>

The contact time during which the salicylic acid solution and the zeolite are brought into contact is not particularly limited as long as impurities are sufficiently removed, but is usually 0.1 hours or more and 24 hours or less.

<Contact pressure>

The contact pressure at which the salicylic acid solution and the zeolite are brought into contact is usually normal pressure, but may be pressurized.

<Contact pH>

The pH of the salicylic acid solution at which the salicylic acid solution and the zeolite are brought into contact is within a range in which salicylic acid does not precipitate, and is usually pH 3 or more and pH 8 or less, from the viewpoint of impurity removal efficiency, preferably pH 3 or more and pH 7 or less, more preferably pH 3 or more and pH 6 or less, and particularly preferably pH 3 or more and pH 5 or less. By setting the pH in the above range, it is possible to efficiently remove at least one or more of the aromatic compounds selected from those represented by the formulas (b), (e), (f), and (g) described below.

(Post-processing)

Examples of the method for isolating the target salicylic acid from the salicylic acid solution obtained through purification step B include a method in which the obtained treated solution is mixed with a strong acid, salicylic acid is precipitated, and crystals of the salicylic acid are obtained by solid-liquid separation (crystallization step described below). The obtained crystals may be further purified by known purification methods such as recrystallization or column chromatography, or by the purification step A described above and/or the purification step C described below.

<Purification step C>

Purification step C is a step to reduce copper ions in salicylic acid and increase the purity of salicylic acid by contacting the salicylic acid obtained in the hydroxylation step or the salicylic acid obtained in the above-mentioned purification step A and/or purification step B with an ion exchange resin.

(Salicylic acid)

The salicylic acid to be purified in the purification step C is usually a solution, preferably an aqueous solution, and a salicylic acid solution obtained in the above-mentioned hydroxylation step or in the above-mentioned purification step B and/or the purification step C can be used.

It is sufficient that the salicylic acid contained in the salicylic acid solution is dissolved when the salicylic acid is in contact with the ion exchange resin. The concentration of salicylic acid in the salicylic acid solution is usually 0.1% by mass or more and 80% by mass or less, preferably 1% by mass or more and 75% by mass or less, and particularly preferably 5% by mass or more and 70% by mass or less, from the viewpoint of productivity and reactivity.

(Ion exchange resin)

As the ion exchange resin used in the purification step C, the above-mentioned ion exchange resins can be used.

(Purification conditions for purification step C)

<Contact temperature>

The contact temperature at which the salicylic acid solution and the ion exchange resin are brought into contact is usually 5°C or higher and 100°C or lower, from the viewpoint of copper ions removal efficiency and solubility, preferably 50°C or more and 95°C or lower, and particularly preferably 60°C or higher and 90°C or lower.

<Contact time>

The contact time during which the salicylic acid solution and the ion exchange resin are brought into contact

is not particularly limited as long as impurities are sufficiently removed, but is usually 0.1 hours or more and 24 hours or less.

<Contact pressure>

The contact pressure at which the salicylic acid solution and the ion exchange resin are brought into contact is usually normal pressure, but may be pressurized.

<pH>

The pH of the salicylic acid solution at which the salicylic acid solution and the ion exchange resin are brought into contact may be within a range in which salicylic acid does not precipitate, and is usually pH 3 or more and pH 8 or less, from the viewpoint of impurity removal efficiency, preferably pH 3 or more and pH 7 or less, more preferably pH 3 or more and pH 6 or less, and particularly preferably pH 3 or more and pH 5 or less. By setting the pH in the above range, it is possible to efficiently remove copper ions.

(Post-processing)

Examples of the method for isolating the target salicylic acid from the salicylic acid solution obtained through purification step C include a method in which the obtained treated solution is mixed with a strong acid, salicylic acid is precipitated, and crystals of the salicylic acid by solid-liquid separation (crystallization step described below). The obtained crystals may be further purified by known purification methods such as recrystallization or column chromatography, or by the purification step A and/or the purification step B described above.

<Crystallization step>

In the crystallization step of the present invention, a pH adjuster is added to the reaction solution containing salicylic acid obtained in the hydroxylation step, or the salicylic acid solution that has undergone one or more of the purification steps A, B, and C described above (hereinafter simply referred to as a salicylic acid solution) to precipitate salicylic acid crystals, which can be recovered by a separation method such as solid-liquid separation.

(pH adjuster)

Examples of the pH adjuster for adjusting the salicylic acid solution include water; acidic pH adjusters such as hydrochloric acid, nitric acid, sulfuric acid, or phosphoric acid and the like; basic pH adjusters such as sodium hydroxide, potassium hydroxide, sodium carbonate, potassium carbonate, sodium hydrogen carbonate, or potassium hydrogen carbonate and the like. Among these, it is preferable to use an acidic pH adjuster, especially sulfuric acid, from the viewpoints of reactivity, productivity, workability, and cost.

The amount of pH adjuster used is not particularly limited, but the amount used should be sufficient to adjust the pH to the above-mentioned pH range.

(Crystallization temperature)

Crystallization temperature refers to the temperature of a solution when salicylic acid is crystallized from a salicylic acid solution. The upper limit of the crystallization temperature is usually 100°C or lower, preferably 95°C or lower, and particularly preferably 90°C or lower, and the lower limit is usually 0°C or higher, preferably 50°C or higher, and particularly preferably 70°C or higher. By setting the crystallization temperature within the above range, impurities can be efficiently removed and salicylic acid having a crystal structure and good filterability can be obtained.

(Crystallization time)

Crystallization time refers to the time required to precipitate salicylic acid from a salicylic acid solution, and is not limited as long as impurities are sufficiently removed, but usually ranges from 0.1 hours or more and 24 hours or less.

(Crystallization pressure)

The crystallization pressure refers to the pressure of the reaction system when salicylic acid is precipitated from a salicylic acid solution, and is usually normal pressure, but may be pressurized.

<pH>

The pH of the salicylic acid solution used for crystallization is within a pH range that suppresses the precipitation of impurities and efficiently precipitates salicylic acid, and is usually pH 0 or more and pH 4 or less, from the viewpoint of impurity removal efficiency, preferably pH 0.5 or more and pH 3.5 or less, and particularly preferably pH 1 or more and pH3 or less. By setting the pH within the above range, salicylic acid precipitates and at least one or more aromatic compounds selected from those represented by the formulas (a), (b), (c) and (d) described below can be removed efficiently.

(Solid-liquid separation temperature)

The solid-liquid separation temperature refers to the temperature of the slurry at the time of solid-liquid separation of salicylic acid crystals after precipitation of salicylic acid, and is usually 0°C or higher and 100°C or lower, from the viewpoint of efficiency of removal of impurities and improvement of yield, preferably 5°C or higher and 40°C or lower, and particularly preferably 10°C or higher and 30°C or lower.

(Solid-liquid separation method)

The method for solid-liquid separation of precipitated salicylic acid crystals is not particularly limited, but in industrial production, solid-liquid separation is usually performed by centrifugation. The salicylic acid crystals obtained by solid-liquid separation can be washed with a solvent in which salicylic acid is difficult to dissolve, such as water.
The filter cloth used for the above-mentioned solid-liquid separation may be any filter cloth that can sufficiently filter out salicylic acid, and any general-purpose filter cloth may be used.

(Drying temperature)

The drying temperature for drying the salicylic acid crystals obtained by solid-liquid separation may be a temperature at which salicylic acid does not sublimate, and is usually 20°C or higher and 40°C or lower.

(Drying pressure)

The drying pressure when drying the salicylic acid crystals obtained by solid-liquid separation may be a pressure that does not sublimate the salicylic acid, and may be either normal pressure or reduced pressure.

(Post-processing)

The crystals obtained by crystallization may be further purified by known purification methods such as recrystallization or column chromatography, or by one or more of the purification steps A, B, and C described above.

[Purification of salicylic acid]

In the method for producing salicylic acid of the present invention, salicylic acid obtained in the aforementioned hydroxylation step may be further purified by a purification process, for example, one or more of the aforementioned purification step A, B, and C or crystallization step, to remove the copper source and by products in the hydroxylation step and mixed into salicylic acid as impurities, specifically, byproducts such as aromatic compounds represented by the following formula (a) to (g). By performing such purification, highly pure salicylic acid can be obtained.
Specifically, high-purity salicylic acid having the HPLC purity of salicylic acid is 95 area % or more and in which the content of each aromatic compounds represented by the following formulas (a) to (g) is 0.5 area % or less can be obtained.

[Chem. 5]

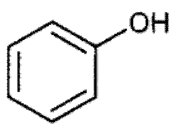
(a)

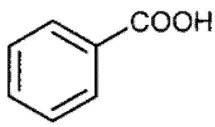
(b)

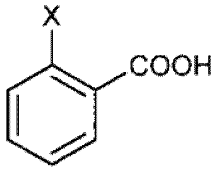
(c)

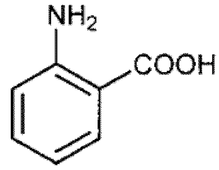
(d)

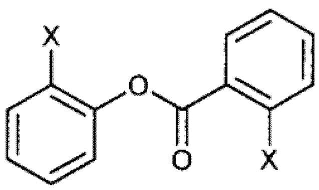
(e)

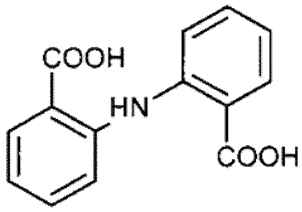
(f)

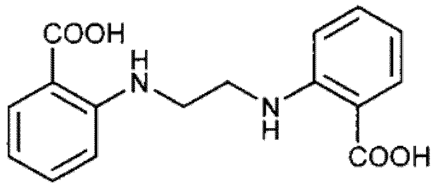
(g)

Aromatic compounds represented by the above formulas (a) to (g) are highly reactive and may cause side reactions in the derivation of salicylic acid to pharmaceuticals, so it is preferable not to allow them to remain in salicylic acid. Furthermore, the aromatic compounds represented by the above formulas (a) to (g) are compounds that are difficult to remove by ordinary purification operations such as solid-liquid separation by crystallization, because their physical properties are close to those of the target product, salicylic acid. It is preferable to remove these compounds prior to solid-liquid separation by crystallization.

The HPLC purity of salicylic acid obtained by the method for producing salicylic acid of the present invention is usually 95 area % or more, preferably 97 area % or more, more preferably 98 area % or more, and particularly preferably 99 area % or more.

When salicylic acid is used as a raw material for pharmaceuticals, the content of the aromatic compounds represented by the above formulas (a) to (g) is respectively usually 0.5 area % or less, preferably 0.4 area % or less, more preferably 0.3 area % or less, even more preferably 0.2 area % or less, and particularly preferably 0.1 area % or less.

Example

The present invention will now be described in more detail with reference to Examples. The present invention is not limited to the following Examples unless it exceeds the gist thereof.

[Calculation of the amount of salicylic acid produced per unit of time]

The amount of salicylic acid produced per unit time, SA/T, which evaluates the productivity of salicylic acid, was calculated according to the following formula.

[Math. 1]

$$SA/T\,[\text{area\%/min.}] = \frac{\text{Salicyluic acid}\ \ [\text{area\%}]}{\text{Reaction time}\ [\text{min.}]}$$

[Abbreviation]

The abbreviations listed below are as follows.

SA : Salicylic acid

| | |
|---|---|
| 2ABA | : 2-aminobenzoic acid (formula (d)) |
| 2CBA | : 2-chlorobenzoic acid (formula (c)) |
| DABA | : 2,2'-iminodibenzoic acid (formula (f)) |
| EDABA | : N,N'-ethylenediaminedianthranilic acid (formula (g)) |
| CuCl | : Copper (I) chloride |
| $CuCl_2$ | : Copper(II) chloride |
| CuBr | : Copper (I) bromide |
| $CuBr_2$ | : Copper(II) bromide |
| CuI | : Copper (I) iodide |
| CuzO | : Copper (I) oxide |
| CuO | : Copper (II) oxide |
| $Cu(OH)_2$ | : Copper (II) hydroxide |
| $Cu(NO_3)_2$ | : Copper (II) nitrate |
| $CuSO_4$ | : Copper (II) sulfate |
| Cu(OAc) | : Copper (I) acetate |
| $Cu(OAc)_2$ | : Copper (II) acetate |
| EDA | : Ethylenediamine |
| L-Pro | : L-Proline |
| N-Me-L-Pro | : N-methyl-L-proline |
| N-Me-L-Pala | : N-methyl-L-alanine |
| L-His | : L-histidine |
| L-Val | : L-valine |
| L-Asp | : L-aspartic acid |
| L-Ser | : L-serine |
| L-Lys | : L-lysine |
| L-Ala | : L-alanine |
| L-Phe | : L-phenylalanine |
| D-Met | : D-methionine |
| EDTA | : Ethylenediaminetetraacetic acid |
| N,N'-DMEDA | : N,N'-dimethylethylenediamine |
| N,N'-TMEDA | : N,N'-tetramethylethylenediamine |

The aromatic compounds represented by the above formulas (a) to (g) are hereinafter referred to as (a) to (g), respectively.

[Flow synthesis reactor]

In the following Examples and Comparative Examples, a flow synthesis reactor shown in Fig. 1 was used.

[Microwave reaction synthesis equipment]

M400 manufactured by Anton Paar
Maximum temperature 300°C, pressure resistance 30bar

[Analysis method (HPLC)]

The apparatus and conditions used to analyze the reaction solution in the following Examples and Comparative Examples are shown in Tables 2A and 2B below.

[Table 2A]

| **< Analysis method 1>** | |
|---|---|
| Analytical equipment | Agilent 1200series<br>manufactured by Agilent Technologies |
| Detector | Diode array detector |

(continued)

| **< Analysis method 1>** | | | |
|---|---|---|---|
| Detection wavelength | 280 nm | | |
| Column | CAPCELL PAK C18 MG3<br>3 μm, 4.6 mmI. D. × 75 mm | | |
| Sample diluted dilution | 50% (v/v) acetonitrile aqueous solution | | |
| Mobile phase A | 0.5% (v/v) phosphoric acid aqueous solution | | |
| Mobile phase B | methanol | | |
| Gradient | Time (minutes) | Mobile phase A (%) | Mobile phase B (%) |
| | 0 | 80 | 20 |
| | 20 | 40 | 60 |
| | 25 | 0 | 100 |
| Flow rate | 1.0 mL/min | | |
| Injection volume | 10 μL | | |
| Column temperature | 40°C | | |
| Analysis time | 25 min | | |
| Retention time | (d): 3.6 min | | |
| | (a): 5.3 min | | |
| | (b): 9.2 min | | |
| | 2CBA: 10.3 min | | |
| | SA: 10.6 min | | |
| | (f): 22.6 min | | |
| | (g): 23.2 min | | |
| | (c): 24.2 min | | |

[Table 2B]

| < Analysis method **2>** | | | |
|---|---|---|---|
| Analytical equipment | Agilent 1100series<br>manufactured by Agilent Technologies | | |
| Detector | UV absorption photometer | | |
| Detection wavelength | **280nm** | | |
| Column | **CAPCELL PAK C18 MG3**<br>**3μm, 4. fimmI. D. × 75mm** | | |
| Sample diluted solution | **50% (v/v)** acetonitrile aqueous solution | | |
| Mobile phase A | **0.5% (v/v)** phosphoric acid aqueous solution | | |
| Mobile phase B | Methanol | | |
| Gradient | Time (minutes) | Mobile phase A **(%)** | Mobile phase B **(%)** |
| | **0** | **80** | **20** |
| | **20** | **40** | **60** |
| Flow rate | **1.0mL/min.** | | |

(continued)

| Gradient | Time (minutes) | Mobile phase A **(%)** | Mobile phase B **(%)** |
|---|---|---|---|
| Injection volume | **10μL** | | |
| Column temperature | **40°C** | | |
| Analysis time | **20 min.** | | |
| Retention time | **(d)** | **:3.6**min. | |
| | **(a)** | **:5.3**min, | |
| | **(b)** | **:9.2**min. | |
| | **2CBA** | **:10.3**min. | |
| | **SA** | **:10.6**min. | |

[Example 1]

780 mg (4.98 mmol) of 2-chlorobenzoic acid, 554 mg (5.24 mmol) of sodium carbonate, 0.49 mg (0.05 mmol, 0.04 mol per 1 mol of 2-chlorobenzoic acid) of copper(I) chloride, and L-proline (0.20 mmol, 0.001 mol per 1 mol of 2-chlorobenzoic acid) were mixed in 3.12 mL of water (4 L per 1 kg of 2-chlorobenzoic acid) at room temperature. The obtained solution was heated to 210°C under microwave using a microwave equipment and reacted for 5 minutes. After the reaction, the reaction solution was cooled to room temperature, and the reaction solution was analyzed by analysis method 1 to obtain salicylic acid at 97.5 area %.

The results of the analysis of the obtained reaction solution by analysis method 1 are shown in Table 3. The obtained reaction solution contained salicylic acid (chemical purity 97.5 area%), and the amount of salicylic acid produced per unit time was 19.5 area%/min.

[Comparative Examples 1 to 3: Reaction temperature]

The reaction was carried out in the same manner as in Example 1, except that the amount of copper (I) chloride used was changed from 0.001 MR to 0.005 MR, and the reaction time and reaction temperature were changed as shown in Table 3. The obtained reaction solution was analyzed in the same manner as in Example 1, and the results are shown in Table 4.

In Table 3, "MR" indicates the number of mols per 1 mol of 2-chlorobenzoic acid. The same applies to the following.

[Table 3]

| | Copper chloride (I) **[MR]** | Reaction temperature **[°C]** | Reaction time [min.] | **2CBA** [ area% ] | **SA** [ area% ] | **SAIT** [area%/min.] |
|---|---|---|---|---|---|---|
| Example 1 | **0.001** | **210** | **5** | **0.2** | **97.5** | **19.5** |
| **Comparative Example 1** | **0.005** | **150** | **30** | **19.2** | **80.5** | **2.1** |
| **Comparative Example 2** | **0.005** | **120** | **30** | **52.3** | **47.4** | **1.6** |
| **Comparative Example 3** | **0.005** | **100** | **300** | **52.7** | **47.3** | **0.2** |

[Examples 2-3: Copper source]

The reaction was carried out in the same manner as in Example 1, except that the copper source was changed as shown in Table 4. The obtained reaction solution was analyzed in the same manner as in Example 1, and the results are shown in Table 4.

[Table 4]

| | copper source | Reaction time [min.] | **2CBA** [ area% ] | **SA** [ area% ] | **SAIT** [area%/min.] |
|---|---|---|---|---|---|
| Example 2 | **$CuCl_2$** | **5** | **7.2** | **91.0** | **18.2** |
| Example 3 | **CuBr** | **5** | **8.5** | **89.8** | **18.0** |

From Examples 2 to 3 in Table 4, it can be seen that salicylic acid can be efficiently obtained even when copper halides other than copper (I) chloride are used as the copper source.

[Examples 4 to 15: Ligand]

The reaction was carried out in the same manner as in Example 1, except that the ligands were changed as shown in Table 5. The obtained reaction solution was analyzed in the same manner as in Example 1, and the results are shown in Table 5.

[Table 5]

| | Ligand | | Reaction time [min.] | **2CBA** [ area% ] | **SA** [ area% ] | **SAIT** [area%/min.] |
|---|---|---|---|---|---|---|
| Example 4 | **L-His** | Amine derivative | **5** | **8.0** | **88.5** | **17.7** |
| Example 5 | **N-Me-L-Pro** | Amine derivative | **5** | **15.0** | **83.0** | **16.6** |
| Example 6 | **L-Val** | Amine derivative | **5** | **16.6** | **81.1** | **16.2** |
| Example 7 | Ethylene diamine | Aliphatic amine | **5** | **18.0** | **79.2** | **15.8** |
| Example 8 | **L-Asp** | Amine derivative | **5** | **19.6** | **78.6** | **15.7** |
| Example 9 | **L-Ser** | Amine derivative | **5** | **20.1** | **76.7** | **15.3** |
| Example 10 | **L-Lys** | Amine derivative | **5** | **23.7** | **73.3** | **14.7** |
| Example 11 | **N-Me-L-Ala** | Amine derivative | **5** | **29.1** | **69.7** | **13.9** |
| Example 12 | **L-Ala** | Amine derivative | **5** | **29.1** | **68.9** | **13.8** |
| Example 13 | **L-Phe** | Amine derivative | **5** | **32.1** | **66.1** | **13.2** |
| Example 14 | **D-Met** | Amine derivative | **5** | **38.4** | **59.3** | **11.9** |
| Example 15 | **EDTA** | Amine derivative | **5** | **56.8** | **42.0** | **8.4** |

From Examples 4 to 15 in Table 5, it can be seen that salicylic acid can be produced with high productivity by using various ligands.

[Example 16]

Salicylic acid was synthesized using the flow synthesis system shown in Fig. 1 that includes a flow-through

reactor provided with a stainless steel tube having an inner diameter of 1.7 mm and a length of 5 m immersed in a temperature-adjustable oil bath.

2-chlorobenzoic acid, sodium carbonate (1.05 mol per 1 mol of 2-chlorobenzoic acid), copper (II) chloride (0.002 mol per 1 mol of 2-chlorobenzoic acid), ethylenediamine (0.08 mol per 1 mol of 2-chlorobenzoic acid) and water (9 L per 1 kg of 2-chlorobenzoic acid) were mixed and dissolved in the preparation tank 1. The obtained solution (hereinafter referred to as 2-chlorobenzoic acid solution) was continuously passed through the flow-through reactor 3 using a back pressure valve to maintain the pump discharge pressure at about 3 MPa so that the reaction time was 5 minutes. Table 6 shows the results of analyzing the reaction product solution obtained by flowing for 30 minutes using the analysis method 1.

The obtained reaction product solution was analyzed by the analysis method 1 and was found to contain salicylic acid (chemical purity 96.5 area %, the amount of salicylic acid produced per unit time: 19.30 area %/min.).

The feed rate of the 2-chlorobenzoic acid solution was maintained at 2.34 mL/min using a plunger pump, and the temperature at the reactor outlet was maintained at 200°C in the flow-through reactor 3.

[Examples 17 to 18 and Comparative Examples 4 to 6: Reaction temperature and reaction time]

The reaction was carried out in the same manner as in Example 16, except that the copper source was changed from copper (II) chloride to copper (I) chloride, the amount used was changed from 0.002 mol to 0.001 mol per 1 mol of 2-chlorobenzoic acid, and the reaction time and reaction temperature were changed shown in Table 6. The obtained reaction solution was analyzed by the analysis method 2 and the results are shown in Table 6.

[Table 6]

| | Reaction temperature **[°C]** | Reaction time [min.] | Analysis results of reaction products [area%] | | | | | **SA/T** [area%/min.] |
|---|---|---|---|---|---|---|---|---|
| | | | **(a)** | **(b)** | **2CBA** | **SA** | Others | |
| Example 16 | **200** | **5** | **0.55** | **0.25** | **0.90** | **96.51** | **1.79** | **19.3** |
| Example 17 | **200** | **2.5** | **0.09** | **0.08** | **64.15** | **35.68** | **0.00** | **14.3** |
| Example 18 | **165** | **2.5** | **0.05** | **0.03** | **74.31** | **25.61** | **0.00** | **10.2** |
| Comparative Example 4 | **140** | **2.5** | **0.00** | **0.00** | **91.46** | **8.54** | **0.00** | **3.4** |
| Comparative Example 5 | **140** | **5** | **0.02** | **0.05** | **76.29** | **23.64** | **0.00** | **4.7** |
| Comparative Example 6 | **140** | **10** | **0.06** | **0.07** | **47.04** | **52.77** | **0.07** | **5.3** |

[Examples 19 to 24: Reaction temperature]

The reaction was carried out in the same manner as in Example 16, except that the amount of water was changed to 18 L per 1 kg of 2-chlorobenzoic acid, and the reaction temperature was set as shown in Table 7. The obtained reaction solution was analyzed by the analysis method 1 and the results are shown in Table 7.

[Table 7]

| | Reaction temperature [°C] | Reaction time [min.] | Analysis results of reaction products [area%] | | | | | | **SA/T** [area%/min.] |
|---|---|---|---|---|---|---|---|---|---|
| | | | **(d)** | **(a)** | **(b)** | **2CBA** | **SA** | Others | |
| Example 19 | **180** | **5** | **0.33** | **0.34** | **0.16** | **1.39** | **97.11** | **0.68** | **19.4** |
| Example 20 | **190** | **5** | **0.33** | **0.50** | **0.13** | **0.08** | **98.11** | **0.85** | **19.6** |
| Example 21 | **195** | **5** | **0.33** | **0.53** | **0.16** | **0.03** | **98.10** | **0.87** | **19.6** |
| Example 22 | **200** | **5** | **0.35** | **0.52** | **0.15** | **0.01** | **98.14** | **0.83** | **19.6** |

(continued)

| | Reaction temperature [°C] | Reaction time [min.] | Analysis results of reaction products [area%] | | | | | | SA/T [area%/min.] |
|---|---|---|---|---|---|---|---|---|---|
| | | | (d) | (a) | (b) | 2CBA | SA | Others | |
| Example 23 | 205 | 5 | 0.35 | 0.51 | 0.15 | 0.05 | 98.05 | 0.89 | 19.6 |
| Example 24 | 210 | 5 | 0.37 | 0.41 | 0.17 | 0.26 | 97.86 | 0.93 | 19.6 |

From Example 1 and Comparative Examples 1 to 3 in Table 3, Examples 16 to 18 and Comparative Examples 4 to 6 in Table 6, and Examples 19 to 24 in Table 7, it can be seen that salicylic acid can be obtained with high selectivity and high productivity in a short reaction time by setting the reaction temperature from 155°C to 300°C.

[Examples 25 to 27 and Comparative Example 7: Amount of ligand used]

The reaction was carried out in the same manner as in Example 16, except that the reaction temperature was 210°C, the length of the stainless steel tube was changed from 5 m to 4 m, and the amount of ligand used was changed as shown in Table 8. The obtained reaction solution was analyzed by the analysis method 1 and the results are shown in Table 8.

[Table 8]

| | Ligand used [MR] | Ligand /Copper source [MR/MR] | Analysis results of reaction products [area%] | | | | | SA/T [area%/min.] |
|---|---|---|---|---|---|---|---|---|
| | | | (a) | (b) | 2CBA | SA | Others | |
| Example 25 | 0.2 | 100 | 0.95 | 0.30 | 0.44 | 94.79 | 3.52 | 19.0 |
| Example 26 | 0.08 | 40 | 0.67 | 0.34 | 2.50 | 94.37 | 2.12 | 18.9 |
| Example 27 | 0.002 | 1 | 0.39 | 0.23 | 55.50 | 42.82 | 1.06 | 8.6 |
| Comparative Example 7 | 0 | 0 | 0.26 | 0.32 | 64.94 | 33.80 | 0.67 | 6.8 |

From Examples 25 to 27 and Comparative Example 7 in Table 8, it can be seen that productivity can be further increased by adding an equivalent or more amount of the ligand to the copper source.

[Examples 28-41: Copper source]

In Example 16, the reaction was carried out in the same manner as in Example 27, except that the reaction temperature was changed to 210°C, the length of the stainless steel tube was changed from 5 m to 4 m, and the reaction temperature and copper source were changed as shown in Table 9. The obtained reaction solution was analyzed by the analysis method 1 and shows the results are shown in Table 9.

[Table 9]

| | Copper source | Analysis results of reaction products [area%] | | | | | SA/T [area%/min.] |
|---|---|---|---|---|---|---|---|
| | | (a) | (b) | 2CBA | SA | Others | |
| Example 28 | Copper(II) citrate · $2.5H_2O$ | 0.81 | 0.43 | 0.37 | 96.09 | 2.31 | 19.2 |
| Example 29 | Copper(II) gluconate | 0.79 | 0.44 | 0.89 | 95.76 | 2.12 | 19.2 |
| Example 30 | Copper(II) nitrate·$3H_2O$ | 0.86 | 0.35 | 1.17 | 95.32 | 2.31 | 19.1 |
| Example 31 | Cu(OAc) | 0.69 | 0.31 | 1.65 | 95.04 | 2.31 | 19.0 |
| Example 32 | $Cu(OAc)_2$ | 0.70 | 0.31 | 1.85 | 94.94 | 2.21 | 19.0 |
| Example 33 | $CuBr_2$ | 0.63 | 0.30 | 2.18 | 94.90 | 1.99 | 19.0 |

(continued)

| | Copper source | Analysis results of reaction products [area%] | | | | | **SA/T** [area%/min.] |
|---|---|---|---|---|---|---|---|
| | | **(a)** | **(b)** | **2CBA** | **SA** | Others | |
| Example 34 | Copper(II) formate·**$3H_2O$** | **0.66** | **0.27** | **2.26** | **94.71** | **2.10** | **18.9** |
| Example 35 | **$Cu(OH)_2$** | **0.65** | **0.30** | **2.26** | **94.62** | **2.18** | **18.9** |
| Example 36 | Copper(II) carbonate | **0.70** | **0.29** | **2.57** | **94.49** | **1.95** | **18.9** |
| Example 37 | **$CuCl_2 \cdot 2H_2O$** | **0.67** | **0.34** | **2.50** | **94.37** | **2.12** | **18.9** |
| Example 38 | **$CuSO_4 \cdot 5H_2O$** | **0.61** | **0.30** | **2.95** | **93.97** | **2.17** | **18.8** |
| Example 39 | **CuI** | **0.70** | **0.29** | **3.95** | **92.86** | **2.19** | **18.6** |
| Example 40 | **CuO** | **0.60** | **0.10** | **25.19** | **72.60** | **1.50** | **14.5** |
| Example 41 | **$Cu_2O$** | **0.52** | **0.11** | **38.04** | **59.80** | **1.54** | **12.0** |

From Examples 2 to 3 in Table 4 and Examples 28 to 41 in Table 9, it can be seen that salicylic acid can be obtained with high selectivity and productivity by using various copper sources.

[Example 42: Base]

The reaction was carried out in the same manner as in Example 16, except that the base was changed from sodium carbonate to potassium carbonate. The obtained reaction solution was analyzed by the analysis method 1 and the result is shown in Table 10 together with the results of Example 16.

[Table 10]

| | Base | Base used **[MR]** | Analysis results of reaction products [area%] | | | | | | **SA/T** [area%/min.] |
|---|---|---|---|---|---|---|---|---|---|
| | | | **(d)** | **(a)** | **(b)** | **2CBA** | **SA** | Others | |
| Example 16 | **$Na_2CO_3$** | **1.05** | **0.36** | **0.55** | **0.25** | **0.90** | **96.51** | **1.43** | **19.3** |
| Example 42 | **$K_2CO_3$** | **1.05** | **0.47** | **0.53** | **0.23** | **0.30** | **97.03** | **1.44** | **19.4** |

From Example 16 and Example 42 in Table 10, it can be seen that salicylic acid can be obtained with high selectivity and high productivity even when an inorganic base other than sodium carbonate is used.

[Examples 43-54: Ligand]

The reaction was carried out in the same manner as in Example 16, except that the ligands were changed as shown in Table 11. The obtained reaction solution was analyzed by the analysis method 1 and the results are shown in Table 11.

[Table 11]

| | Ligand | | Analysis results of reaction products [area%] | | | | | | Others | **SA/T** [area%/min.] |
|---|---|---|---|---|---|---|---|---|---|---|
| | | | **(d)** | **(a)** | **(b)** | **2CBA** | **SA** | **(f)** | | |
| Example 43 | N,N'-dimethylcyclohexane-1,2-diamin | Aliphatic amine | **0.00** | **0.62** | **0.28** | **0.03** | **98.19** | **0.00** | **0.88** | **19.6** |
| Example 44 | **N,N'-DMEDA** | Aliphatic amine | **0.15** | **0.91** | **0.15** | **0.00** | **97.89** | **0.001** | **0.90** | **19.6** |
| Example 45 | **N,N'-DMEDA** | Aliphatic amine | **0.34** | **0.88** | **0.25** | **0.00** | **97.25** | **0.11** | **1.16** | **19.4** |
| Example 46 | Iminodiacetic acid | Amine derivative | **0.04** | **0.62** | **0.61** | **1.02** | **96.67** | **0.00** | **1.04** | **19.3** |
| Example 47 | **N,N'-TMEDA** | Aliphatic amine | **0.10** | **0.92** | **0.46** | **3.73** | **93.58** | **0.02** | **1.20** | **18.7** |
| Example 48 | Triethylenetetramine | Aliphatic amine | **0.62** | **1.33** | **1.03** | **0.89** | **89.88** | **2.55** | **3.69** | **18.0** |
| Example 49 | 1,3-propyldiamine | Aliphatic amine | **0.34** | **0.76** | **0.46** | **9.60** | **84.34** | **1.54** | **2.96** | **16.9** |
| Example 50 | Spermine | Aliphatic amine | **0.68** | **0.88** | **0.97** | **11.48** | **81.40** | **1.93** | **2.67** | **16.3** |
| Example 51 | Spermidine | Amine derivative | **1.08** | **0.92** | **1.96** | **12.41** | **75.03** | **5.73** | **2.87** | **15.0** |
| Example 52 | Dimethylamine | Aliphatic amine | **0.08** | **0.77** | **1.05** | **22.07** | **74.39** | **0.00** | **1.65** | **14.9** |
| Example 53 | Diethylamine | Aliphatic amine | **0.00** | **0.69** | **0.65** | **36.69** | **61.39** | **0.07** | **0.50** | **12.3** |
| Example 54 | Bipyridyl | Heterocyclic amine | **0.00** | **0.46** | **0.14** | **55.05** | **43.84** | **0.00** | **0.51** | **8.8** |

From Examples 4 to 15 in Table 5 and Examples 43 to 54 in Table 11, it can be seen that salicylic acid can be obtained with high selectivity and high productivity by using amines such as aliphatic amines, heterocyclic amines, and amine derivatives or the like as ligands.

(Example 55)

The reaction was carried out in the same manner as in Example 16, except that the amount of water used was increased from 9 times (9 L) to 18 times (18 L) the amount of 2-chlorobenzoic acid. The obtained reaction solution was analyzed by the analysis method 1 and the result shown in Table 12.

[Examples 56 to 64: Adsorbent (synthetic adsorbent)]

1 mL of the reaction solution obtained in Example 55 was adjusted to pH 3.7 with 50% by mass sulfuric acid, and about 10 mg (20% by mass based on 2-chlorobenzoic acid) of the adsorbent listed in Table 12 (synthetic adsorbent manufactured by Mitsubishi Chemical Corporation) was added and shaken at 35°C and 1000 rpm for 5 hours using a shaker (TS100C manufactured by BIOSAN Ltd.). The obtained purified salicylic acid solution was analyzed by analysis method 1 and the results are shown in Table 12.

[Table 12]

| | Synthetic adsorbent | | | Analysis results of purified salicylic acid solution [area%] | | | |
|---|---|---|---|---|---|---|---|
| | Product name | Base material | Pore diameter [nm] | **SA** | **(f)** | **(g)** | **(e)** |
| Example 55 | - | - | - | **96.79** | **0.07** | **0.11** | **0.13** |
| Example 56 | **HP21** | Styrene-divinylbenzene copolymer | **11** | **97.59** | **0.02** | **0.00** | **0.01** |
| Example 57 | **SP70** | Styrene-divinylbenzene copolymer | **7** | **97.45** | **0.02** | **0.06** | **0.02** |
| Example 58 | **SP850** | Styrene-divinylbenzene copolymer | **5** | **97.42** | **0.00** | **0.10** | **0.02** |
| Example 59 | **HP20SS** | Styrene-divinylbenzene copolymer | **29** | **97.39** | **0.02** | **0.02** | **0.02** |
| Example 60 | **SP700** | Styrene-divinylbenzene copolymer | **9** | **97.37** | **0.01** | **0.02** | **0.01** |
| Example 61 | **SP207** | Bromostyrene-divinylbenzene copolymer | **11** | **97.35** | **0.01** | **0.05** | **0.03** |
| Example 62 | **HP20** | Styrene-divinylbenzene copolymer | **29** | **97.33** | **0.04** | **0.01** | **0.01** |
| Example 63 | **HP2MG** | Methyl methacrylate-bis (methacrylic acid) ethyleneglycol copolymer | **24** | **97.17** | **0.00** | **0.04** | **0.02** |
| Example 64 | **HP2MGL** | Methyl methacrylate-bis (methacrylic acid) ethyleneglycol copolymer | **24** | **97.03** | **0.01** | **0.01** | **0.03** |

From Examples 56 to 64 in Table 12, it can be seen that impurities can be efficiently removed by using a synthetic adsorbent.

[Examples 65 to 66 and Comparative Example 8: Adsorbent (synthetic adsorbent)]

1 mL of the reaction solution obtained in Example 55 was adjusted to pH 3.7 with 50% by mass sulfuric acid, and about 10 mg (20% by mass based on 2-chlorobenzoic acid) of the adsorbent listed in Table 13 (synthetic adsorbent manufactured by Mitsubishi Chemical Corporation) was added and shaken at 35°C and 1000 rpm for 5 hours using a shaker (TS100C manufactured by BIOSAN Ltd.). The obtained purified salicylic acid solution was analyzed by analysis method 1 and the results are shown in Table 13.

[Table 13]

| | Synthetic adsorbent | | | Analysis results of purified salicylic acid solution [area%] | | | | |
|---|---|---|---|---|---|---|---|---|
| | Product name | Base material | Pore diameter [nm] | **(b)** | **SA** | **(f)** | **(g)** | **(a)** |
| Example 55 | - | - | - | **0.18** | **96.79** | **0.07** | **0.11** | **0.13** |
| Example 65 | **XAD4** | Styrene-divinylbenzene copolymer | **12** | **0.15** | **97.64** | **0.04** | **0.00** | **0.00** |
| Example 66 | **XAD1600N** | Styrene-divinylbenzene copolymer | **21** | **0.14** | **97.41** | **0.00** | **0.11** | **0.00** |
| Comparative Example 8 | **XAD761** | Phenol-formalin copolymer | **60** | **0.15** | **96.92** | **0.06** | **0.12** | **0.08** |

From Examples 65 to 66 in Table 13, it can be seen that impurities can be efficiently removed by using a styrene-based synthetic adsorbent.

[Examples 67 to 70: Adsorbent (activated carbon)]

15 mL of the reaction solution obtained in Example 16 was adjusted to pH 3.5 with 50% by mass sulfuric acid, and about 10 mg (10% by mass based on 2-chlorobenzoic acid) of the adsorbent listed in Table 14 (activated carbon manufactured by Osaka Gas Chemicals Co., Ltd.) was added and stirred at 75°C for 5 hours. The obtained purified salicylic acid solution was analyzed by the analysis method 1 and the results are shown in Table 14.

[Table 14]

| | Activated carbon | Analysis results of purified salicylic acid solution [area%] | | | |
|---|---|---|---|---|---|
| | | **SA** | **(f)** | **(g)** | **(e)** |
| Example 67 | - | **96.84** | **0.14** | **0.05** | **0.09** |
| Example 68 | Seisei Shirasagi | **97.82** | **0.02** | **0.00** | **0.00** |
| Example 69 | Kyoryoku Shirasagi | **97.68** | **0.02** | **0.00** | **0.00** |
| Example 70 | Shirasagi | **97.64** | **0.03** | **0.00** | **0.00** |

From Examples 67 to 70 in Table 14, it can be seen that impurities can be efficiently removed by using activated carbon.

[Example 71 and Comparative Examples 9 to 12: Adsorbent (zeolite)]

1 mL of the solution obtained in Example 55 was adjusted to pH 3.7 with 50% by mass sulfuric acid, and about 10 mg (20% by mass based on 2-chlorobenzoic acid) of the adsorbent listed in Table 15 (zeolite manufactured by TOSOH Corporation) was added and shaken at 35°C and 1000 rpm for 5 hours using a shaker (TS100C manufactured by BIOSAN Ltd.). The obtained purified salicylic acid solution was analyzed by the analysis method 1 and the results are shown in Table 15.

[Table 15]

| | Zeolite | | | Analysis results of purified salicylic acid solution [area%] | | | | |
|---|---|---|---|---|---|---|---|---|
| | Product name | $SiO_2/Al_2O_3$ | Pore diameter [nm] | (b) | SA | (f) | (g) | (e) |
| Example 55 | - | - | - | 0.18 | 96.79 | 0.07 | 0.11 | 0.13 |
| Example 71 | HSZ-891HOA | 1880 | 5.8 | 0.04 | 97.12 | 0.06 | 0.03 | 0.09 |
| Comparative Example 9 | HS-341 NH4 | 7 | 2-4 | 0.15 | 96.97 | 0.08 | 0.04 | 0.14 |
| Comparative Example 10 | HS-500K | 6 | 2-4 | 0.16 | 96.85 | 0.06 | 0.03 | 0.08 |
| Comparative Example 11 | HS-320H | 5.5 | 6 | 0.17 | 97.14 | 0.05 | 0.05 | 0.12 |
| Comparative Example 12 | HS-320Na | 5.5 | 6 | 0.17 | 96.88 | 0.10 | 0.04 | 0.14 |

From Example 71 and Comparative Examples 9 to 12 in Table 15, it can be seen that by using zeolite having a high $SiO_2/Al_2O_3$ ratio, the aromatic compound represented by the formula (b) can be selectively removed.

[Example 72 and Comparative Examples 14 to 16: Adsorbent (ion exchange resin)]

A column (5 mm × 100 mm) filled with ion exchange resin shown in Table 16 (manufactured by Mitsubishi Chemical Corporation) (0.90 g) and water was set in a column type flow reactor (manufactured by EYELA, model: MCR-1000 type). 90 g of the reaction solution obtained in Example 55 was passed through the above column at a rate of 0.9 mL/min to obtain a purified salicylic acid solution. The results of ICP analysis of the copper ion content of the obtained purified salicylic acid solution are shown in Table 16.

[Table 16]

| | Ion exchange resin | | Copper ion content [ppm] |
|---|---|---|---|
| | Product name | Functional group | |
| Example 55 | - | - | 83 |
| Example 72 | CR11 | Iminodiacetic acid group | 25 |
| Comparative Example 14 | SK1B | Sulfonic acid group | 74 |
| Comparative Example 15 | PK216 | Sulfonic acid group | 76 |
| Comparative Example 16 | CRB03 | N-methylglucamine group | 80 |

From Example 72 and Comparative Examples 14 to 16 in Table 16, it can be seen that copper ions can be efficiently adsorbed and removed by using an ion exchange resin having a chelate type ion exchange resin such as iminodiacetic acid group.

[Example 73]

(i) Hydroxylation step

Sodium carbonate (1.05 molar equivalents to 2-chlorobenzoic acid), 2-chlorobenzoic acid, copper(II) chloride (0.002 molar equivalents to 2-chlorobenzoic acid), and ethylenediamine (0.08 molar equivalents to 2-chlorobenzoic acid) were mixed and dissolved in an aqueous solution (water; 18 L for 1 kg of 2-chlorobenzoic acid) and supplied to a coil reactor and allowed to flow. In this step, the supply rate of the mixed solution was set to 2.34 mL/min (reaction time: 5

minutes) using a plunger pump. The reaction was carried out in the coil reactor while it was immersed in an oil bath set so that the temperature of the reaction solution was 190°C.

After 30 minutes of flow-through (44.7 g of 2-chlorobenzoic acid as the starting material was used in the reaction), 873.7 g of the resulting reaction solution was analyzed by analysis method 1, and as a result, it contained 97.7 area% of salicylic acid.

(ii) Purification step I

A column (10 mm x 100 mm) filled with ion exchange resin DIAION (registered trademark) CR11 (4.06 g) and water was set in a column type flow reactor (manufactured by EYELA, model: MCR-1000 type). 860 g of the reaction solution obtained above was passed through the above column at a rate of 2.87 mL/min to obtain 865.7 g of purified salicylic acid solution I.

(iii) Purification step II

196.5 g of the obtained purified salicylic acid solution I was heated to 75°C using a 200 mL separable flask, and the pH was adjusted to 3.5 using 50% by mass sulfuric acid. A synthetic adsorbent DIAION (registered trademark) HP21 (20 mass% based on 2-chlorobenzoic acid used in the reaction) was added to the solution, and after stirring for 2 hours, the solution was filtered to remove the synthetic adsorbent to obtain purified salicylic acid solution II.

[Example 74: Crystallization pH]

94.7 g of purified salicylic acid solution II obtained in Example 73 was heated to 85°C, and 50% by mass sulfuric acid was added dropwise over 1 hour until the pH reached 2.6. The solution was then cooled to 25°C at a rate of 5°C/hour. After cooling, filtration was carried out to obtain 2.4 g of salicylic acid crystals. The obtained salicylic acid crystals were analyzed by analysis method 1 and the results are shown in Table 17.

[Example 75: Crystallization pH]

190.0 g of purified salicylic acid solution II obtained in Example 73 was heated to 85°C, and 50% by mass sulfuric acid was added dropwise over 1 hour until the pH reached 1.8. The solution was then cooled to 25°C at a rate of 10°C/hour. After cooling, filtration was carried out to obtain 8.0 g of salicylic acid crystals. The obtained salicylic acid crystals were analyzed by analysis method 1 and the results are shown in Table 17.

[Example 76: Crystallization pH]

92.1 g of purified salicylic acid solution II obtained in Example 73 was heated to 85°C, and 50% by mass sulfuric acid was added dropwise over 1 hour until the pH reached 0.9. The solution was then cooled to 25°C at a rate of 10°C/hour. After cooling, filtration was carried out to obtain 4.3 g of salicylic acid crystals. The obtained salicylic acid crystals were analyzed by analysis method 1 and the results are shown in Table 17.

[Table 17]

| | Crystallization temperature [°G] | pH | Result of analysis [area%] | | | | | |
|---|---|---|---|---|---|---|---|---|
| | | | (d) | (a) | (b) | 2CBA | SA | Others |
| Example 73 | - | 3.5 | 0.33 | 0.43 | 0.15 | 0.07 | 98.80 | 0.22 |
| Example 74 | 85 | 2.6 | 0.00 | 0.00 | 0.10 | 0.00 | 99.87 | 0.03 |
| Example 75 | 85 | 1.8 | 0.00 | 0.00 | 0.06 | 0.01 | 99.86 | 0.07 |
| Example 76 | 85 | 0.9 | 0.00 | 0.00 | 0.05 | 0.00 | 99.86 | 0.09 |

From Examples 74-76 in Table 17, it can be seen that impurities can be efficiently removed by precipitating salicylic acid at a relatively low pH.

[Example 77]

(i) Hydroxylation step

Sodium carbonate (1.05 molar equivalents to 2-chlorobenzoic acid), 2-chlorobenzoic acid, copper(II) chloride (0.001 molar equivalents to 2-chlorobenzoic acid), and N,N'-dimethylethylenediamine (0.04 molar equivalents to 2-chlorobenzoic acid) were mixed and dissolved in an aqueous solution (water; 9 L volume for 1 kg of 2-chlorobenzoic acid) and supplied to a coil reactor and allowed to flow. In this step, the supply rate of the mixed solution was set to 2.34 mL/min (reaction time: 5 minutes) using a plunger pump. The reaction was carried out in the coil reactor while it was immersed in an oil bath set so that the temperature of the reaction solution was 200°C.

After 30 minutes of flow-through (38.0 g of 2-chlorobenzoic acid as starting material was used in the reaction), 393.9 g of the resulting reaction solution was analyzed by analysis method 1, and as a result, it contained 99.4 area% of salicylic acid.

(ii) Purification step I

Column A (10 mm x 100 mm) filled with ion exchange resin DIAION (registered trademark) CR11 (3.80 g) and water was set in a column type flow reactor (manufactured by EYELA, model: MCR-1000 type). 393.9 g (38.0 g as 2-chlorobenzoic acid) of the reaction solution obtained in (i) was passed through column A at a rate of 2.87 mL/min, and 401.5 g (38.0g as 2-chlorobenzoic acid) of purified salicylic acid solution I was obtained.

(iii) Purification step II

Column B (5 mm x 200 mm) filled with synthetic adsorbent DIAION (registered trademark) HP21 (2.24 g) and water was set in a column type flow reactor (manufactured by EYELA, model: MCR-1000 type). 180.5 g (16.8 g as 2-chlorobenzoic acid) of purified salicylic acid solution I obtained in (ii) was passed through column B at a rate of 0.64 mL/min, and 191.1 g (16.8 g as 2-chlorobenzoic acid) of purified salicylic acid solution II was obtained.

(iv) Purification step III

95.0 g (8.4 g as 2-chlorobenzoic acid) of the purified salicylic acid solution II obtained in (iii) was heated to an internal temperature of 75°C using a 200 mL separable flask, and the pH was adjusted to 3.6 using 50% by mass sulfuric acid. Zeolite (HSZ-891HOA manufactured by Tosoh Corporation, 20% by mass based on the 2-chlorobenzoic acid used in the reaction) was added to the pH-adjusted solution, and after stirring for 1 hour, the zeolite was removed by filtration and 88.7 g of purified salicylic acid solution III was obtained.

(v) Crystallization step

88.7 g of the purified salicylic acid solution III obtained in (iv) was heated to an internal temperature of 85°C, and then 50% by mass sulfuric acid was added dropwise over 1 hour to adjust the pH to 1.8. The pH-adjusted solution was cooled from 85°C to 25°C at a cooling rate of 10°C/hr. After cooling, the solution was filtered to obtain 6.4 g of salicylic acid crystals (yield: 89.9%). The salicylic acid crystals were analyzed by analysis method 1, and the salicylic acid purity was 99.95 area %.

Industrial applicability

According to the method for producing salicylic acid of the present invention, salicylic acid useful as a pharmaceutical intermediate can be produced from 2-halogenated benzoic acid at low cost and with high productivity. The obtained salicylic acid is industrially useful because it can be used as a raw material for pharmaceuticals or intermediates thereof such as methyl salicylate.

Although the present invention has been described in detail with reference to particular embodiments, it will be apparent to those skilled in the art that various modifications may be made therein without departing from the spirit and scope of the present invention.

The present application is based on Japanese Patent Application No. 2021-139134 filed August 27, 2021, which is incorporated herein by reference in its entirety.

Reference Signs List

1 preparation tank
2 pump
3 flow synthesis reactor
4 collection tank
5 back pressure valve

## Claims

1. A method for producing salicylic acid comprising a hydroxylation step in which salicylic acid is produced by reacting 2-halogenated benzoic acid in an aqueous solvent at a reaction temperature of 155°C or higher and 300°C or lower in the presence of a copper source, a ligand, and a base.

2. The method for producing salicylic acid according to claim 1, wherein a reaction pressure of the reaction is 0.1 MPa or more and 10 MPa or less.

3. The method for producing salicylic acid according to claim 1 or 2, wherein the ligand is a compound having one or more amino groups which is optionally substituted.

4. The method for producing salicylic acid according to any one of claims 1 to 3, wherein the copper source is a copper compound.

5. The method for producing salicylic acid according to any one of claims 1 to 4, comprising a purification step B in which the salicylic acid produced in the hydroxylation step is brought into contact with zeolite.

6. The method for producing salicylic acid according to any one of claims 1 to 5, comprising a purification step A in which the salicylic acid produced in the hydroxylation step is brought into contact with a synthetic adsorbent.

7. The method for producing salicylic acid according to any one of claims 1 to 6, wherein the reaction is a flow synthesis reaction.

8. The method for producing salicylic acid according to claim 6 or 7, the salicylic acid from the purification step A or the purification step B has HPLC purity of 95 area % or more, and contains one or more aromatic compounds represented by the following formulas (a) to (g) as impurities, and the content of each aromatic compound is 0.5 area % or less.

[Chem. 1]

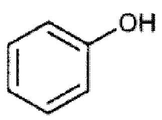

(a)

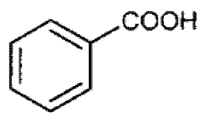

(b)

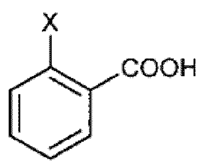

(c)

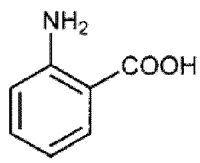

(d)

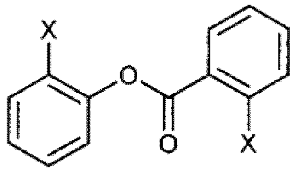

(e)

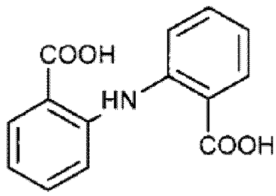

(f)

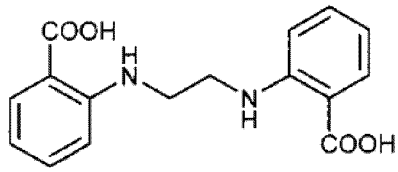

(g)

[Fig. 1]

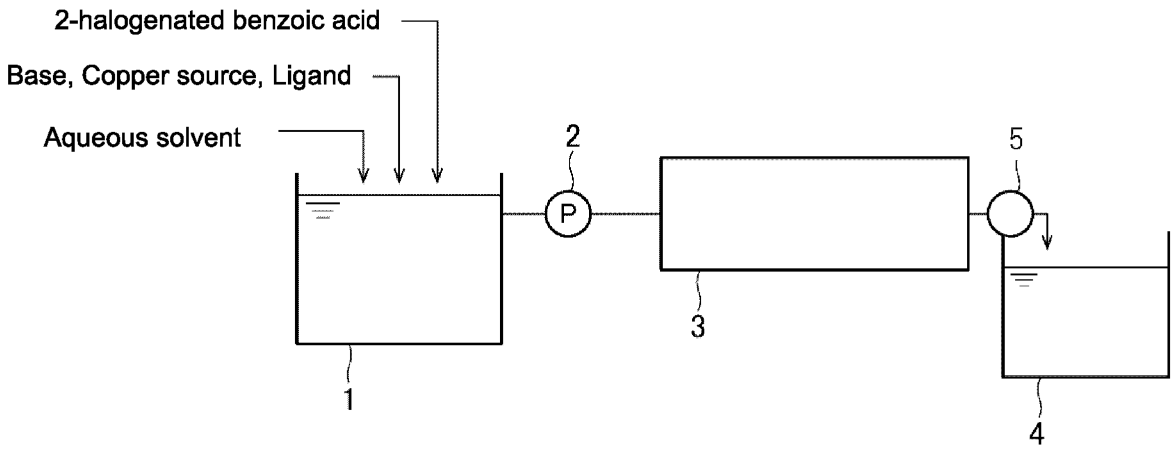
2-halogenated benzoic acid
Base, Copper source, Ligand
Aqueous solvent
2
P
5
3
1
4

## INTERNATIONAL SEARCH REPORT

International application No. **PCT/JP2022/032190**

**A. CLASSIFICATION OF SUBJECT MATTER**

***C07C 51/367***(2006.01)i; ***C07B 61/00***(2006.01)i; ***C07C 51/47***(2006.01)i; ***C07C 65/10***(2006.01)i
FI: C07C51/367; C07B61/00 300; C07C51/47; C07C65/10

According to International Patent Classification (IPC) or to both national classification and IPC

**B. FIELDS SEARCHED**

Minimum documentation searched (classification system followed by classification symbols)

C07C51/367; C07B61/00; C07C51/47; C07C65/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY /CASREACT(STN)

**C. DOCUMENTS CONSIDERED TO BE RELEVANT**

| Category* | Citation of document, with indication, where appropriate, of the relevant passages | Relevant to claim No. |
|---|---|---|
| A | JP 2010-511043 A (E. I. DU PONT DE NEMOURS AND CO.) 08 April 2010 (2010-04-08) claims 1-22, paragraph [0033], examples 1, 2, 4-6, 30-32 | 1-8 |
| A | KE, F. et al. Microwave-assisted copper-catalyzed hydroxylation of aryl halides in water. RSC Advances. 2013, vol. 3, pp. 22837-22840 table 2, entry 16 | 1-8 |
| A | PALACIOS, M. L. Docampo et al. Synthesis of Salicylic Acid Derivatives in Presence of Ultrasonic Irradiation Using Water as Solvent. Synthetic Communications. 2003, vol. 33, no. 10, pp. 1783-1787 Results and discussion, tables 1-3 | 1-8 |
| A | JP 55-53240 A (NIPPON KAYAKU CO., LTD.) 18 April 1980 (1980-04-18) claims, p. 2, lower left column, lines 6-10, examples 1-6 | 1-8 |
| A | JP 2001-64230 A (NISSAN CHEM. IND., LTD.) 13 March 2001 (2001-03-13) claims 2-8, examples 5-11 | 1-8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 October 2022** | **08 November 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/032190**

| Patent document cited in search report | Publication date (day/month/year) | Patent family member(s) | Publication date (day/month/year) |
|---|---|---|---|
| JP 2010-511043 A | 08 April 2010 | US 2008/0139843 A1<br>claims 1-22, paragraph [0031], examples 1, 2, 4-6, 30-32<br>WO 2008/066820 A1<br>EP 2099736 A1<br>KR 10-2009-0083947 A<br>CN 101541731 A | |
| JP 55-53240 A | 18 April 1980 | (Family: none) | |
| JP 2001-64230 A | 13 March 2001 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S5815939 A
- JP 2010511043 A
- JP 2021139134 A

### Non-patent literature cited in the description

- *SYNTHETIC COMMUNICATIONS,* 2002, vol. 32 (13), 2055-2059